# EUROPEAN PATENT APPLICATION

(11) **EP 3 964 523 A1**
(43) Date of publication of application: **09.03.2022**
(21) Application number: 20020398.2
(22) Date of filing: 04.09.2020
(51) Int. Cl.: C07K 14/81, C07K 1/20, A61K 38/17, A61K 38/57

(54) **PREPARATION OF FETUIN A BY HYDROPHOBIC INTERACTION CHROMATOGRAPHY**

(71) Applicant: PreviPharma Consulting GmbH, 68167 Mannheim (DE)
(72) Inventor: Kießig, Stephan T., D-69168 Wiesloch (DE); Berger, Tina, D-64625 Bensheim (DE); Mandago, Maurice, D-69250 Schönau (DE)
(74) Representative: Sacht-Gorny, Gudrun

(57) **Abstract**

The invention provides a method for preparing Fetuin A from a Fetuin A-containing source material, especially from a biological liquid. The method comprises the steps of providing a Fetuin A containing source material, loading the Fetuin A containing source material on a hydrophobic interaction chromatography material, wherein Fetuin A adsorbs to the chromatography material, and eluting Fetuin A.

## Description

The present invention relates to a method for preparing Fetuin A (alpha-2-HS glycoprotein) from a biological liquid. Moreover, the present invention relates to a protein preparation comprising Fetuin A, and a pharmaceutical composition comprising such a protein preparation.

Fetuin A is also known as alpha-2-HS glycoprotein and belongs to the fetuin class of plasma-binding proteins which manage transport and availability of various substances in the bloodstream. There are further synonyms for Fetuin A respectively alpha-2-HS glycoprotein, namely alpha-2-HS, A2HS, AHS and HSGA.

Fetuins occur in large quantities in fetal blood. The most prominent representative of the fetuin class of proteins is serum albumin. There are two further types of fetuins, namely Fetuin A and Fetuin B. Normal serum concentrations of Fetuin A in healthy adults range between 0.3 and 1.0 g/L.

Fetuin A is mainly expressed in the liver, but also in the kidney and in choroid plexus. Fetuin A is highly expressed in the non-collagenous bone matrix during fetal life and affects bone growth via antagonizing the effects of transforming growth factor beta (Schlieper G., Westenfeld R., Brandenburg V., Ketteler M., Inhibitors of calcification in blood and urine. Semin Dial 2007, 20: 113-121; Heiss A., DuChesne A., Denecke B., Grotzinger J., Yamamoto K., Renne T., Jahnen-Dechent W., Structural basis of calcification inhibition by alpha 2-HS glycoprotein/fetuin-A. Formation of colloidal calciprotein particles. J Biol Chem 2003, 278: 13333-13341; Quelch K.J., Cole W.G., Melick R.A., Non-collagenous proteins in normal and pathological human bone. Calcif Tissue Int 1984; 36: 545-549).

Lack of Fetuin A has been shown to result in severe systemic calcification in mice and humans (Ketteler M., Bongartz P., Westenfeld R., Wildberger J., Mahnken A., Bohm R., Metzger T., Wanner C., Jahnen-Dechent W., Floege J., Association of low fetuin-A (AHSG) concentrations in serum with cardiovascular mortality in patients on dialysis: a cross-sectional study. Lancet 2003; 361: 827-833; Reynolds J.L., Skepper J.N., McNair R., Kasama T., Gupta K., Weissberg P.L., Jahnen-Dechent W., Shanahan C.M., Multifunctional roles for serum protein fetuin-a in inhibition of human vascular smooth muscle cell calcification. J Am Soc Nephrol 2005, 16: 2920-2930).

Fetuin A facilitates the removal of hydroxyapatite crystals by increasing the calciprotein particles in the bloodstream and, therefore, inhibiting intravascular calcification (Heiss A. et al., see above).

The bone is a living, active tissue constantly remodeling itself through bone resorption and formation. An imbalance between the two stages leads to osteoporosis. Osteoporotic bones are spongier (porous) and have lower density or mass and contain abnormal tissue structure. Consequently, when bones become weak and an increased risk of spontaneous fractures (hip, spine or wrist) occurs. The basic framework of bones is given by collagen fibers. The minerals (calcium, phosphate) are responsible for hardening the bones. In both, men and women, osteoporosis onset with advanced age (in women 5 - 7 years after menopause). The first symptoms are permanent pain, losing height, stooped or hunched posture, limiting mobility often leading to isolation or depression. The current standard of care is based on a basic therapy using calcium, phosphate and vitamin D in combination with other drugs. Also the bone resorption can be targeted by slowing medications (e.g. bisphosphonate, estrogen, or specific monoclonal antibodies acting on osteoclasts). Sometimes bone building promoting drugs (e.g. fluorides, or parathyroid hormones) may increase the activity of bone-building cells (osteoblasts).

Nevertheless, the current therapy is not always sufficient to prevent vertebral and bone fractures. One main limitation in the currently used drugs is their lower efficacy on non-vertebral fracture reduction than on vertebral fracture. Moreover, there are severe side effects and potential adverse events on long-term use. Additionally, the treatment of osteoporosis and of related bone fractures is very cost intensive.

In general, people with age of ≥ 50 years have a high risk of osteoporotic bone fractures. In 2010 158 million people worldwide suffered from osteoporosis, this number will be almost doubled up to 2040 to 310 million individuals. Nearly half of all women are expected to suffer an osteoporotic fracture in their lifetime (Sweet MG, Sweet JM, Jeremiah MP, Galazka SS. Diagnosis and treatment of osteoporosis. Am Fam Physician 2009; 79: 193-200). There are 7 - 20 million people expected in 2050 only with osteoporosis-related hip fractures. Twenty percent of the patients with hip fracture become functionally dependent and require long-term nursing care (Jaglal SB, Sherry PG, Schatzker J. The impact of hip fracture in Ontario and its consequences. Can J Surg 1996; 2: 105-111).

The exact mechanism of Fetuin A in bone mineralization has not been completely elucidated. In the course of an *in vitro* rat study, Toroian et al. noted that Fetuin A stimulated calcification within bone (Toroian D., Lim J.E., Price P.A., The size exclusion characteristics of type I collagen: implications for the role of non-collagenous bone constituents in mineralization. J Biol Chem 2007, 282(31): 22437-22447). There are limited human studies on Fetuin A to define its exact mechanism (Ix J.H., Wassel C.L., Bauer D.C., Toroian D., Tylavsky F.A., Cauley J.A., Harris T.B., Price P.A., Cummings S.R., Shlipak M.G., Fetuin-A and bone mineral density in older persons: The Health Aging and Body Composition (Health ABC) study. J Bone Miner Res 2009, 24: 514-521; Chailurkit L., Kruavit A., Rajatanavin R., Ongphiphadhanakul B., The relationship of fetuin-A and lactoferrin with bone mass in elderly women. Osteoporos Int 2011; 22: 2159-2164).

Fetuin A is commercially available, especially prepared from fetal calf serum. It is known to prepare Fetuin A by ammonium sulfate precipitation or by cold ethanol precipitation according to Spiro (Spiro R. G., Journal of Biological Chemistry 235, 10: 2860, 1960). Moreover, a one-step affinity purification of Fetuin from fetal bovine serum was developed on the basis of wheat germ agglutinin affinity separation (Cartellieri S. et al., Biotechnol. Appl. Biochem. 2002, 35 (2): 83 - 9). The preparation from fetal serum is widely preferred because the content of Fetuin A in fetal plasma is much higher than in adult plasma.

There is still a need for effective preparation methods for Fetuin A from biological liquids, especially from human plasma, preferably from adult plasma. The present invention has the object to provide an efficient preparation method for Fetuin A from biological liquids and especially from plasma or a plasma-derived preparation or from other sources. The fetuin in plasma is often strongly bound to albumin in a way, that Fetuin A can be prepared only as an Fetuin A / Albumin complex. For pharmaceutical purposes it would be preferable to provide an albumin-free Fetuin A preparation.

This object is solved by a method as depicted in claim 1. Preferred embodiments are outlined in the dependent claims. Furthermore, the object is solved by a protein preparation and a pharmaceutical composition as outlined in the further independent and dependent claims.

The inventive method for preparing Fetuin A (alpha-2-HS glycoprotein) from a Fetuin A containing source material, e.g. a biological liquid, comprises the following steps:
- providing a Fetuin A containing source material,
- loading the Fetuin A containing source material on a hydrophobic interaction chromatography material, wherein Fetuin A adsorbs to the chromatography material, and
- eluting Fetuin A.

Surprisingly, the inventors found out that Fetuin A can be prepared very effectively by hydrophobic interaction chromatography (HIC). The resulting Fetuin A fractions are highly pure and basically free of further impurities. A major advantage of the inventive method is the very effective separation of albumin from the preparation.

The Fetuin A containing source material may be any material which contains Fetuin A, especially a biological liquid like blood, serum, and, mostly preferred, plasma or a plasma fraction.

The loading step is done under conditions where Fetuin A binds respectively adsorbs to the chromatography material, especially under high-salt conditions. Preferably after a washing step the elution is performed, wherein the hydrophobic interactions are weakened, especially by a decreasing salt gradient. As found by the inventors, such hydrophobic interaction chromatography is unexpectedly useful for preparing Fetuin A from biological fluids and plasma fractions.

The general principle of hydrophobic interaction chromatography is based in hydrophobic interactions between the target protein and the chromatography material. By this method the proteins or peptides are separated according to their affinity to hydrophobic groups immobilized to an insoluble matrix. The sample which contains molecules respectively proteins with hydrophobic and hydrophilic regions are applied to the chromatography material. The salt in the buffer reduces the solvation of sample solutes. As solvation decreases, hydrophobic regions that become exposed are adsorbed by the chromatography material. The more hydrophobic the protein, the less salt is needed to promote binding. For elution of the proteins preferably a decreasing salt gradient is used to elute the proteins from the chromatography material in order of increasing hydrophobicity.

Generally, the hydrophobic interaction chromatography material comprises a support and one or more immobilized ligands. In preferred embodiments of the inventive method the hydrophobic interaction chromatography material comprises an alkyl ligand and/or an aryl ligand linked to a polymeric support. In general, the straight chain alky ligands show the most hydrophobic character. The aryl ligands show a mixture of aromatic and hydrophobic behaviour.

In especially preferred embodiments of the inventive method the alkyl ligand and/or the aryl ligand is at least one ligand chosen from the group consisting of methyl, butyl, butyl-S (S - sulfur), phenyl, octyl, ether and isopropyl.

In mostly preferred embodiments the ligand is octyl or butyl. It has been shown by the inventors that especially by these ligands Fetuin A can be very effectively prepared.

In preferred embodiments the support of the hydrophobic interaction chromatography is cross-linked agarose like Sepharose or synthetic copolymer materials.

In an especially preferred embodiment of the inventive method the hydrophobic interaction chromatography material is Octyl-Sepharose.

In another preferred embodiment the hydrophobic interaction chromatography material contains mildly hydrophobic ligands like t-butyl (t - tertiary).

Preferably, the ligand is characterized by the following formula:

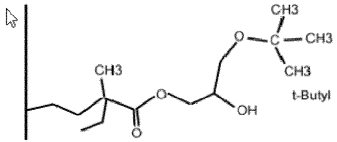

Preferably, the support, i.e. the base matrix composition, is a resin of metacrylate-based beads with a particle size of preferably 30 - 80 µm, more preferably 50 µm ± 10 µm. Preferably, the hydrophobic interaction chromatography material is t-butyl linked to methacrylate-based beads. A particularly preferred example of the hydrophobic interaction chromatography material is Macro-Prep T-Butyl HIC Resin (Bio-Rad).

In an alternative embodiment of the inventive method the hydrophobic interaction chromatography material is a hydrophobic ion exchange material, preferably an aromatic hydrophobic ion exchange material, more preferably an aromatic hydrophobic cation exchange material (also designated as a mixed mode resin). As has been shown by the inventors also such a hydrophobic interaction chromatography material may be used to efficiently prepare Fetuin A with a high purity. In an especially preferred embodiment, the hydrophobic ion exchange material is an aromatic hydrophobic cation exchange material. Preferably, the hydrophobic cation exchange material is a weak cation exchange material.

Preferably, the ligand of the aromatic hydrophobic cation exchange material is characterized by the following formula:

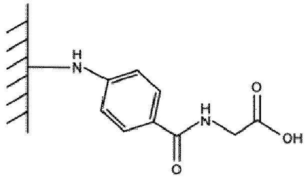

Preferably, the support, i.e. the base matrix composition, is a microporous highly crosslinked polymer with a particle size of preferably 50 - 100 µm, more preferably 70 µm ± 10 µm. In an especially preferred embodiment of the inventive method a Foresight Nuvia^{™} cPrime^{™} column (Bio-Rad) is used as a hydrophobic cation exchange resin material.

The chromatography step of the inventive method may be performed as column chromatography or batch chromatography. Especially for larger scale purposes like industrial purposes column chromatography may be preferred.

In preferred embodiments the Fetuin A-containing source material and especially the biological liquid is plasma or a plasma-derived preparation, especially human plasma or a human plasma-derived preparation. Nevertheless, the inventive method may be used to prepare Fetuin A from other sources, e.g. from bovine or plasma from other species. Other sources may be other body fluids or plasma fractions containing Fetuin A or, e.g., cell culture supernatants from cells expressing Fetuin A naturally or based on recombinant techniques.

It is especially preferred to integrate the inventive method into usually practiced fractionation processes of human plasma in order to use the limited raw material plasma for the preparation of different plasma proteins including the preparation of Fetuin A according to the inventive method.

The inventors were able to show that Fetuin A can be purified in a very efficient way from a Cohn fraction or a Kistler-Nitschman fraction (Kistler-Nitschman is a variant of the Cohn process), especially from Cohn fraction IV. The Cohn fractionation process is a widely established plasma fractionation process which has been developed in 1946, wherein the Cohn process involves a series of precipitation steps using different ethanol concentrations and varying temperatures and pH values. The process is based on the differential solubility of the diverse plasma proteins under the different conditions. Cohn fraction IV is achieved by 40% ethanol at pH 5.8 at low temperature. The dissolved precipitate (paste) of this step (Cohn fraction IV) or its subfractions may be preferably used as source material for the inventive method. In a preferred embodiment Cohn fraction IV or its subfractions may be used without the removal of albumin or before or after removal albumin.

In especially preferred embodiments the Fetuin A-containing source material, especially Cohn IV fraction or its subfractions, is used without removal of albumin, because the use of the hydrophobic interaction chromatography according to the inventive method efficiently separates albumin from Fetuin A.

It is especially preferred to use high-salt concentrations when equilibrating and loading the hydrophobic interaction chromatography material in the course of the inventive method. The addition of salts promotes the ligand-protein interactions in hydrophobic interaction chromatography.

Especially the loading step is performed at high-salt, preferably at 1 to 2 M ammonium sulfate ((NH₄)₂SO₄), more preferably at 1.5 M ammonium sulfate. Alternatively, 1 to 2 M sodium chloride (NaCI), preferably 1.5 M NaCl, may be used. Other salts like potassium thiocyanate (KSCN), sodium sulfate (Na₂SO₄), potassium chloride (KCI), ammonium acetate (CH₃COONH₄) can be used alternatively. Moreover, the salt concentration can be varied between 300 - 8000 mM. Especially preferred is a molarity of 1.5 M salt.

Preferably, the high salt concentration is adjusted in the loading and equilibration buffer as well as in the loading sample itself. It is a main advantage of the inventive method that a high salt concentration allows the dissociation of the Albumin / Fetuin A complex.

In preferred embodiments, before loading the chromatography material with the biological liquid, the chromatography material is equilibrated to a high salt concentration of 1.5 M ammonium sulfate or another salt like NaCl.

The elution of the adsorbed Fetuin A is preferably performed by decreased ionic strength. In an especially preferred embodiment of the inventive method the elution step is performed at a decreasing salt gradient or with a decreased salt concentration, e.g. a stepwise decreased salt concentration. A decreasing salt gradient is especially preferred in combination with column chromatography. The hydrophobic interactions of the target molecule with the hydrophobic interaction chromatography material are gradually reduced by the decreasing salt concentration.

The decreasing salt gradient results in a specific separation of albumin from Fetuin A on the chromatography material. In preferred embodiments albumin is found in the flow-through. The desorbed Fetuin A can be eluted with a high degree of purity. When analyzing the elution fractions Fetuin A appears as a strong band at about 60 kDa and a weaker band at about 50 kDa on SDS-PAGE as verified by Western Blotting.

An especially preferred example for a decreasing NaCl gradient is e.g. a gradient from 1500 mM up to 10 mM NaCl. The inventors were able to achieve very good results with such an NaCl gradient. Nevertheless, it is also possible to run a stepwise elution with a decreased salt concentration. In especially preferred embodiments Fetuin A may be eluted in the range of 1500 mM - 10 mM NaCl with a high purity.

The buffering ions are generally not critical for hydrophobic interaction, but important for stabilization of pH value, selectivity, and resolution of hydrophobic interaction chromatographic separation. Preferably, a pH between 4 - 8.5 is used for the separation, preferably pH 5 - 8.5, more preferably pH 6 - 8, even more preferably pH 7.

Ions could be e.g. sodium phosphate, sodium acetate, sodium dihydrogen phosphate (NaH₂PO₄), Tris/HCI, MES or potassium phosphate in a concentration range between 10 to 300 mM. 50 - 100 mM sodium phosphate buffer at pH 7 is especially preferred, most preferably 100 mM sodium phosphate butter, pH 7.

Preferably, all buffers used for the chromatography step have the same pH, e.g. pH 4 - 8, especially the buffers used for equilibration, charging, loading, washing and elution. It has been shown by the inventors that especially under such conditions, especially at pH 7, a purification of Fetuin A with very good results can be achieved. Preferably, during the whole chromatography step including loading, optionally washing and the following elution the pH value is not essentially changed.

In preferred embodiments of the inventive method the chromatography step is performed in the presence of at least one stabilizer for prevention of degradation of Fetuin A, wherein, preferably, the at least one stabilizer is a charged cation, e.g. a monovalent cation like Na⁺, K⁺ and/or Li⁺. In further preferred embodiments a doubly charged cation may be used as a stabilizing agent, e.g. Ca²⁺ and/or Mg²⁺. Moreover, other agents like EDTA, DTT, glycerol, alcohols, detergents, chaotropic salts (MgCl₂, CaCl₂, KJ, Na-isothiocyanate, K-isothiocyanate, urea) can be used to improve the chromatographic separation.

In principle, it is also possible to use small amounts of albumin or other proteinaceous stabilizers, e.g. up to 5 % (w/v) albumin. Nevertheless, it may be preferred to not us any proteinaceous stabilizers in order to provide a highly pure product without side-proteins.

In more detail the inventive method for preparation of Fetuin A may comprise the following steps:
(i) providing blood plasma or a plasma fraction comprising Fetuin A, preferably any Cohn fraction IV,
(ii) contacting the blood plasma or the plasma fraction with the hydrophobic interaction chromatography material (HIC material), e.g. by loading a column filled with the chromatography material under high-salt conditions,
(iii) washing the HIC material obtained from step (ii) with a first buffer (washing buffer with 1500 mM salt concentration),
(iv) eluting the Fetuin A from the washed HIC material with a second buffer (elution buffer with low salt concentration) thereby depressing hydrophobic interactions by decreased salt concentrations with the HIC material, wherein preferably the cations may be applied by a salt gradient, preferably a NaCl gradient or a ammonium sulfate gradient using a minimal concentration of e.g. 10 mM NaCl or 10 mM ammonium sulfate in a second buffer for elution.

Optionally, during the chromatography Fetuin A may be stabilized by adding one or more stabilizers that prevent the protein from degradation. The stabilizers may be added at the elution step or later. The one or more stabilizers which may be optionally added are preferably charged cations as outlined above.

The pH value in the solution obtained from the elution step may be adjusted to a desired range or value. Preferably, the pH is kept constant during the chromatography, preferably near neutral.

In preferred embodiments the purification may be combined with one or more antiviral treatments. Especially the solution obtained from the elution step may be subjected to one or more antiviral treatments as they are known to an expert in the art. Examples of antiviral treatments are solvent/detergent treatments, UV irradiation, nanofiltration, heat treatment, especially dry heat treatment or others. E.g. the optional antiviral treatment may be performed by adding one or more detergents, preferably one or more detergents selected from the group consisting of Tween-20, Tween-80 and Triton-X-100. Additionally, or alternatively, one or more other antiviral agents such as a phosphate ester, in particular tri-n-butyl-phosphate (TNBP) may be added. Preferably, a solvent/detergent treatment may be applied. Moreover, filtration steps may be applied like ultrafiltration and, especially preferred, nanofiltration. In especially preferred embodiments combinations of two or more of the aforementioned antiviral treatments may be applied.

Moreover, the obtained product may be provided in a solid, especially dried form, wherein the method may comprise drying or freeze-drying of the eluted Fetuin A directly obtained from elution or obtained from further optional steps after elution.

The inventors were able to show that the described method enables to obtain comparably high yields of rather pure Fetuin A from different plasma fractions, especially from plasma fractions which may be obtained from commonly used plasma fractionation processes. In comparison to prior art preparation processes for Fetuin A, the method of the present invention enables to obtain increased protein yields and to minimize degradation or denaturation of Fetuin A with a high overall protein purity. Typically, the inventive method allows a purity of more than 60% (w/w) in relation to the total polypeptide mass of the obtained preparation, preferably the purity is 80% (w/w) or even greater. Preferably, the obtained product is free from any serum albumin and IgG, IgM, and IgA.

It is an especially advantageous feature of the inventive method that the method may be integrated into existing plasma fractionation processes. The source material of the inventive method, namely the loading material, may be plasma or plasma intermediates, e.g. cryoprecipitate or cryo-poor plasma or a fraction or waste fraction of a precipitation step in plasma fractionation like the Cohn process or the Kistler-Nitschmann process (a variant of the Cohn process), as long, of course, the material contains Fetuin A. Cryo-poor plasma is typically obtained from the supernatant of blood plasma subjected to being frozen and subsequently thawn. In especially preferred embodiments the waste fraction(s) in the Cohn or Kistler-Nitschmann process of paste I + II + III or waste fraction(s) of paste I + III of the Cohn or Kistler-Nitschmann process or a combination of two or all three of these fractions may be used. Moreover, paste I + II + III or paste I + III of the Cohn or Kistler-Nitschmann process or any fraction or waste fraction thereof containing Fetuin A may be used. In especially preferred embodiments paste IV of the Cohn or Kistler-Nitschmann process or any subfraction, fraction or waste fraction thereof containing Fetuin A may be used as a source material for loading the chromatography material according to the inventive method.

The flow-through of the chromatography step of the inventive method may flow directly into further fractionation steps, like further steps of the Cohn process, for example.

Moreover, the invention comprises a protein preparation comprising Fetuin A obtainable or obtained by the described process. Preferably, the preparation comprises at least 60% (w/w), more preferably at least 80% (w/w) Fetuin A in relation to total polypeptide mass of the preparation.

Preferably, the protein preparation is free from serum albumin and/or Ceruloplasmin and/or transferrin and/or ferritin and/or IgG and/or IgM and/or IgA and/or haptoglobin, for example. Preferably, the protein preparation is free from serum albumin. More preferably, the protein preparation is free from serum albumin and IgG and IgM and IgA. Preferably, the immunoglobulins are already removed in the load material due to the preceding Cohn fractionation process when using especially paste IV for providing the Fetuin A containing source material. Serum albumin is effectively removed by the hydrophobic interaction chromatography as described above. The inventors were able to show by non-reducing SDS-PAGE that the obtained product in the elution fractions is highly pure. Moreover, an analysis for albumin by a Bromcresol Green Assay showed that albumin is below the detection limit (< 1.25 g/L) in the eluted fractions.

Moreover, the invention comprises a pharmaceutical composition comprising a protein preparation as described above and at least one pharmaceutically acceptable carrier and/or excipient. The protein preparation of this pharmaceutical composition is obtainable by the inventive method as described above and may be further characterized by an advantageously high purity of Fetuin A, especially a purity of at least 60% (w/w), preferably at least 80% (w/w), in relation to the whole polypeptide or protein content of the composition.

The pharmaceutically acceptable excipient or carrier may be a suitable excipient or carrier like, e.g., buffers, stabilizing agents, bulking agents, a preservative or the like as it is known to an expert in the art. The pharmaceutical composition may be a liquid (solution or gel or the like) or in dried form, e.g., lyophilized. If the pharmaceutical composition is in lyophilized form, the composition may be intended for reconstitution with a suitable liquid, e.g. with water or an appropriate buffer. The pharmaceutical composition may be intended for different administration routes, e.g. for oral application in the form of tablets, pills, powders, or the like, or for intravenous or subcutaneous application or intraperitoneal or intramuscular administration routes.

Moreover, the invention comprises a pharmaceutical composition comprising a protein preparation comprising Fetuin A as described above and at least one pharmaceutically acceptable carrier for use in treatment or prophylaxis in patients suffering from Fetuin A deficiency. Generally, the Fetuin A deficiency is independent from the cause of the deficiency, namely inborn or acquired Fetuin A deficiency. According to this aspect of the invention patients suffering from Fetuin A deficiencies may be treated or prophylactically treated with the pharmaceutical composition comprising Fetuin A. Thus, Fetuin A respectively alpha-2-HS glycoprotein according to this invention may be used also to treat patients suffering from acquired or inborn Fetuin A deficiencies in general. Those patients may therefore be at risk to develop an osteoporosis with a high risk for bone fractures.

In an especially preferred embodiment the protein preparation comprising Fetuin A as component of the pharmaceutical composition is obtained by the above-defined preparation method of Fetuin A. Preferably, the Fetuin A is a human plasma protein, prepared from a human biological fluid, preferably prepared from human plasma or a human plasma-derived preparation. Nevertheless, it is also comprised that the Fetuin A is not a human protein. It may be a recombinant or synthetic Fetuin A based on human or non-human genetic information or it may be a native Fetuin A from another species, or recombinant protein e.g., Fetuin A respectively alpha-2-HS glycoprotein may be a bovine protein.

In especially preferred embodiments Fetuin A is prepared according to the above described method from native sources, like plasma or plasma-derived preparations, e.g. human plasma or human plasma-derived preparations or other body fluids. Moreover, it may be especially preferred to prepare a recombinant Fetuin A, which may be produced and preferably secreted by recombinant cells in cell culture. Thus, it may be especially preferred to prepare Fetuin A according to the inventive method by the use of corresponding cell culture supernatants obtained from recombinant cells which produce Fetuin A and which are prepared by genetic engineering techniques. The cells may be prokaryotic cells like, e.g., *Escherichia coli* or other commonly used cells in recombinant techniques, or insect cells, or, especially preferred, eukaryotic cells, like Chinese hamster ovarian cells (CHO) or human cell lines like HEK (Human Embryonic Kidney) cells, which may be used for recombinant protein production.

The pharmaceutical composition of the present invention is preferably used for those patients who are characterized by a decreased level of Fetuin A, which is preferably lower than 90% confidence interval of healthy subjects (90% confidence interval: 0.0094 ... 0.2716 g/L in the chosen control population) in comparison. Additionally, or alternatively, the level of Fetuin A in the blood of the patient is at least 1% (mol/mol) lower in comparison to the average level found throughout a comparable population of the same species.

In especially preferred embodiments of the described pharmaceutical compositions the pharmaceutical composition is for use in treatment or prophylaxis in patients suffering from osteoporosis and/or in patients on risk of bone fractures and/or in patients on risk of hyper-calcification and/or in patients on risk of endochondral extraosseous or heterotopic ossification. Thus, the invention comprises a method for treating a patient suffering from or being at risk of developing a disorder of the bone metabolism in terms of osteoporosis and calcinosis.

In the figures it is shown:
- Fig. 1: Chromatogram of the inventive method using an Octyl-Sepharose column;
- Fig. 2A: SDS-PAGE (non-reducing conditions) after purification via Octyl-Sepharose column;
- Fig. 2B: Western Blot (non-reducing conditions) after purification via Octyl-Sepharose column;
- Fig. 3: Chromatogram of the inventive method using an t-Butyl-HIC column;
- Fig. 4A: SDS-PAGE (non-reducing conditions) after purification via t-Butyl-HIC column;
- Fig. 4B: Western Blot (non-reducing conditions) after purification via t-Butyl-HIC column;
- Fig. 5: Chromatogram of the inventive method using a mixed mode resin (cPrime) column;
- Fig. 6A: SDS-PAGE (non-reducing conditions) after purification via mixed mode resin (cPrime) column; and
- Fig. 6B: Western Blot (non-reducing conditions) after purification via mixed mode resin (cPrime) column.

### Examples

### Example 1: Method of production of a Fetuin A preparation (alpha-2-HS glycoprotein) on HiTrap^{®} Octyl-Sepharose Fast Flow Column

For purification of Fetuin A (alpha-2-HS glycoprotein) from a plasma derived preparation a HiTrap^{®} Octyl-Sepharose Fast Flow Column (GE Healthcare Life Sciences, Germany, Article No.GE17-5196-01) with a column volume (CV) of 5 mL has been used. The chromatography was performed at room temperature.

### Run condition:

- Load: 13 mL (2.6 CV) feed paste IV (in 0.1 M sodium phosphate, 1.5 M ammonium sulfate, pH 7)
- DF: 16.02 mL (3.26 CV)
- Wash: 75 mL (15 CV)
- Elution: 100 mL (20 CV) gradient and 50 mL (10 CV) isocratic
- Eluate fractions: A19 - B6

### Buffers:

- Equilibration + Wash: 0.1 M sodium phosphate, 1.5 M ammonium sulfate, pH 7
- Elution: 0.1 M sodium phosphate, pH 7 → gradient 20 CV and 10 CV isocratic

### Results:

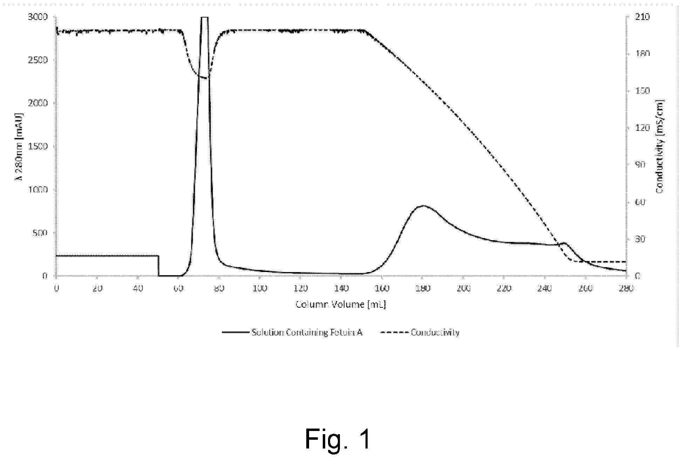

The resulting chromatogram is shown in **Fig. 1****.** The x-axis indicates the volume in mL, the left y-axis indicates the protein content as measured by OD₂₈₀- The right y-axis indicates the conductivity in mS/cm. Fetuin A was found at a high purity in the eluted fractions A19 - B6 (corresponding to volume 158 mL - 273 mL).

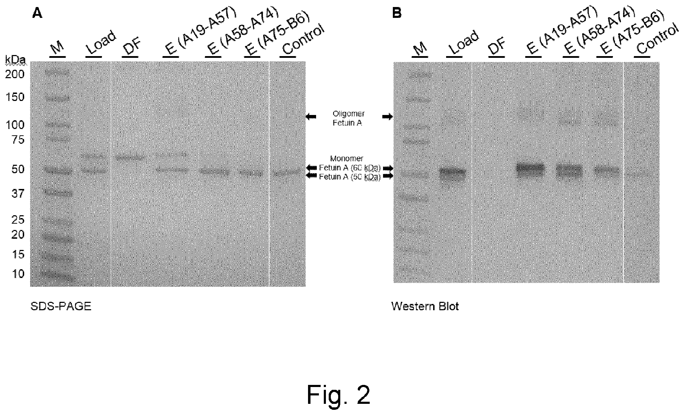

The resulting SDS-PAGE (under non-reducing conditions) according to Laemmli is shown in **Fig. 2A** (M - marker, DF - flow-through, E - eluate). A Western Blot using a polyclonal anti-Fetuin A antibody (BioVendor GmbH, Germany) is shown in **Fig. 2B** (M - marker, DF - flow-through, E - eluate). As can be seen from the SDS-PAGE and the Western Blot the eluate fractions contain Fetuin A at a high purity. Serum albumin (about 66 kDa) ist found in the flow-through.

### Example 2: Method of production of a Fetuin A preparation on Macro-Prep t-Butyl HIC Column

For purification of Fetuin A from a plasma derived preparation a Macro-Prep t-Butyl HIC Column (Bio-Rad, US, Article No. 1580090) with a column volume (CV) of 5.6 mL has been used. The chromatography was performed at room temperature.

### Run condition:

- Load: 13 mL (2.32 CV) feed paste IV (in 0.1 M sodium phosphate, 1.5 M ammonium sulfate, pH 7)
- DF: 16.02 mL (2.86 CV)
- Wash: 86 mL (15.36 CV)
- Elution: 11.2 mL (2 CV) gradient and 56 mL (10 CV) isocratic
- Eluat fractions: A12 - A28

### Buffers:

- Equilibration + Wash: 0.1 M sodium phosphate, 1.5 M ammonium sulfate, pH 7
- Elution: 0.1 M sodium phosphate, pH 7 → gradient 2 CV and 10 CV isocratic

### Results:

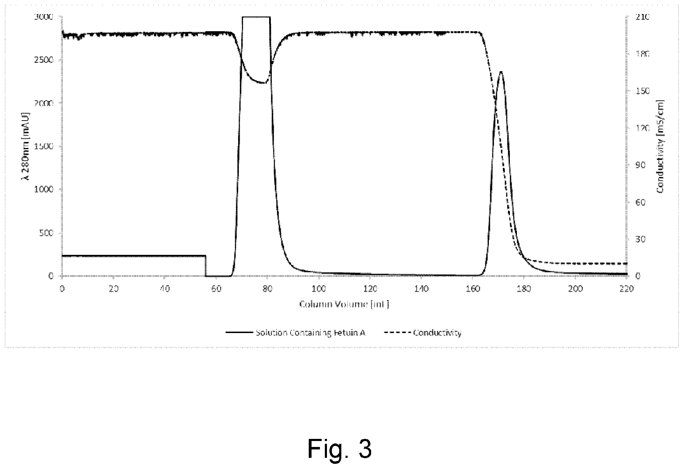

The resulting chromatogram is shown in **Fig. 3****.** The x-axis indicates the volume in mL, the left y-axis indicates the protein content as measured by OD₂₈₀. The right y-axis indicates the conductivity in mS/cm. Fetuin A was found at a high purity in the eluted fractions A12 - A28 (corresponding to volume 165 mL - 180 mL).

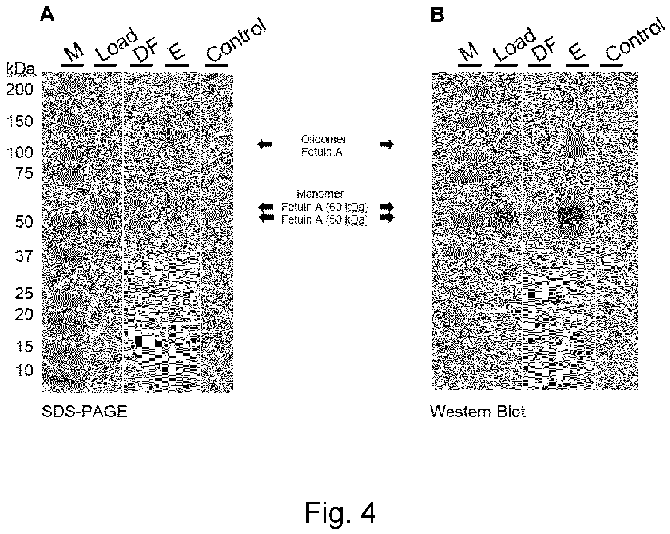

The resulting SDS-PAGE (under non-reducing conditions) according to Laemmli is shown in **Fig. 4A** (M - marker, DF - flow-through, E - eluate). A Western Blot using a polyclonal anti-Fetuin A antibody (BioVendor GmbH, Germany) is shown in **Fig. 4B** (M - marker, DF - flow-through, E - eluate). As can be seen from the SDS-PAGE and the Western Blot the eluate fractions contain Fetuin A at a high purity.

### Example 3: Method of production of a Fetuin A preparation on Mixed Mode Resin

For purification of Fetuin A (alpha-2-HS glycoprotein) from a plasma derived preparation a Foresight Nuvia^{™} cPrime^{™} column for immobilized hydrophobic cation exchanger (Bio-Rad, US, Article No. 732- 4742) with a column volume (CV) of 5 mL has been used. The chromatography was performed at room temperature.

### Run condition:

- Load: 12 mL (2.4 CV) feed paste IV (in 0.1 M sodium phosphate, 1.5 M ammonium sulfate, pH 7)
- DF: 15.02 mL (3.004 CV)
- Wash: 75 mL (15 CV)
- Elution: 100 mL (20 CV) gradient and 75 mL (15 mL) isocratic
- Eluat fractions: A19 - A70

### Buffers:

- Equilibration + Wash: 0.1 M sodium phosphate, 1.5 M ammonium sulfate, pH 7
- Elution: 0.1 M sodium phosphate pH 7 → gradient 20 CV and 15 CV isocratic

### Results:

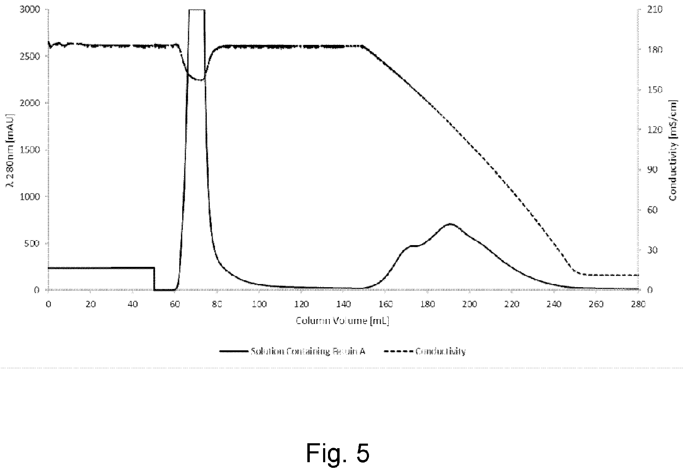

The resulting chromatogram is shown in **Fig. 5****.** The x-axis indicates the volume in mL, the left y-axis indicates the protein content as measured by OD₂₈₀. The right y-axis indicates the conductivity in mS/cm. Fetuin A was found at a high purity in the eluted fractions A19 -A70.

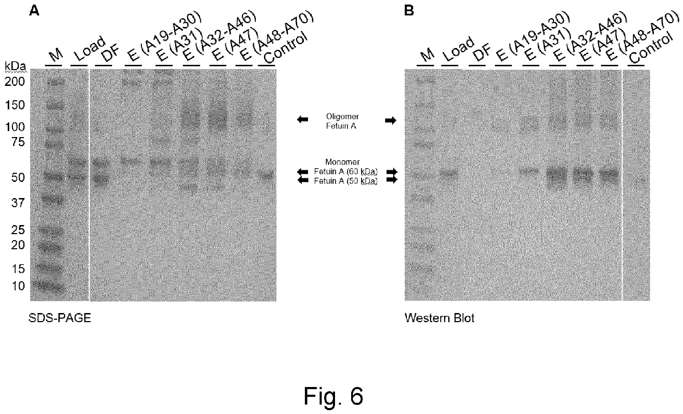

The resulting SDS-PAGE (under non-reducing conditions) according to Laemmli is shown in **Fig. 6A** (M - marker, DF - flow-through, E - eluate). A Western Blot using a polyclonal anti-Fetuin A antibody (BioVendor GmbH, Germany) is shown in **Fig. 6B** (M - marker, DF - flow-through, E - eluate). As can be seen from the SDS-PAGE and the Western Blot the eluate fractions contain Fetuin A at a high purity, wherein the highest purity is found in the later fractions A32 - A70.

## Claims

1. Method for preparing Fetuin A from a Fetuin A containing source material, especially a biological liquid, comprising the following steps:
- providing a Fetuin A containing source material,
- loading the Fetuin A containing source material on a hydrophobic interaction chromatography material, wherein Fetuin A adsorbs to the chromatography material, and
- eluting Fetuin A.

2. Method of claim 1, wherein the hydrophobic interaction chromatography material comprises an alkyl ligand and/or an aryl ligand linked to a polymeric support.

3. Method of claim 2, wherein the alkyl ligand and/or the aryl ligand is at least one ligand chosen from the group consisting of methyl, butyl, butyl-S, phenyl, octyl, ether and isopropyl.

4. Method of claim 2 or claim 3, wherein the ligand is octyl or butyl.

5. Method of one of the preceding claims, wherein the hydrophobic interaction chromatography material is Octyl-Sepharose.

6. Method of one of the preceding claims, wherein the hydrophobic interaction chromatography material is t-butyl linked to methacrylate-based beads.

7. Method of one of claims 1 to claim 4, wherein the hydrophobic interaction chromatography material is a hydrophobic ion exchange material, preferably an aromatic hydrophobic ion exchange material.

8. Method of claim 7, wherein the hydrophobic ion exchange material is an aromatic hydrophobic cation exchange material.

9. Method of one of the preceding claims, wherein the Fetuin A-containing source material is a plasma-derived material.

10. Method of claim 9, wherein the plasma-derived material is Cohn fraction IV or Cohn fraction IV before or after removal of albumin.

11. Method of one of the preceding claims, wherein the loading step is performed at high-salt, preferably at 1 to 2 M ammonium sulfate, more preferably at 1.5 M ammonium sulfate.

12. Method of one of the preceding claims, wherein the elution step is performed at a decreasing salt gradient.

13. Method of one of the preceding claims, wherein the chromatography step is performed in the presence of at least one stabilizer for prevention of degradation of Fetuin A, wherein, preferably, the at least one stabilizer is a doubly charged cation.

14. Protein preparation comprising Fetuin A obtainable by a process according to one of the claims 1 to 13, wherein the preparation comprises at least 60%, preferably at least 80% (w/w) Fetuin A.

15. Pharmaceutical composition comprising a protein preparation according to claim 14 and at least one pharmaceutically acceptable carrier and/or excipient.
